# EUROPÄISCHE PATENTSCHRIFT

(11) **EP 1 699 508 B1**
(45) Veröffentlichungstag und Bekanntmachung des Hinweises auf die Patenterteilung: **21.11.2007**
(21) Anmeldenummer: 04765451.2
(22) Anmeldetag: 21.09.2004
(51) Int. Cl.: A61M 5/158

(54) **INSERTIONSVORRICHTUNG FüR DURCHSTECHVORRICHTUNGEN**
INSERTION UNIT FOR PUNCTURE DEVICES
DISPOSITIF D'INSERTION POUR DISPOSITIFS DE PERFORATION

(30) Priorität: 30.12.2003 DE 20320207 U
(43) Veröffentlichungstag der Anmeldung: 13.09.2006
(73) Patentinhaber: Disetronic Licensing AG, 3401 Burgdorf (CH)
(72) Erfinder: LINIGER, Jürg, CH-3072 Ostermundigen (CH); ROETHLISBERGER, Marc, CH-3415 Hasle-Rueegsau (CH); SCHEURER, Simon, CH-3006 Bern (CH)
(74) Vertreter: Schalch, Rainer
(86) Internationale Anmeldenummer: PCT/EP2004/010575
(87) Internationale Veröffentlichungsnummer: WO 2005/065748

(56) Entgegenhaltungen:
- US-A- 4 085 748
- US-A1- 2003 060 781
- US-B1- 6 293 925

## Beschreibung

Die vorliegende Erfindung betrifft eine Insertionsvorrichtung für Infusionssets.

Infusionssets sind zum Beispiel aus US 6302866 bekannt. Insertionsvorrichtungen zum Einbringen von Infusionssets sind zum Beispiel aus US 6293925 bekannt. Solche Insertionsvorrichtungen weisen einen an der Innenmantelfläche des Gehäuses entlang gleitenden Kolben auf, welcher über eine Aufnahmevorrichtung zur Aufnahme des Infusionssets verfügt. US 2003/0060781 A1 zeigt verschiedene Insertionsvorrichtungen für Infusionssets, bei denen das Infusionsset an einem Vortriebselement befestigt wird, welches zum Applizieren des daran befestigten Infusionssets am Körper eines Patienten durch die Kraft einer vorgespannten Feder in Einstechrichtung bewegt wird.

Aufgabe der Erfindung ist es, eine Insertionsvorrichtung zu schaffen, die es erlaubt, die Energie zum Einbringen eines Infusionssets möglichst verlustfrei, insbesondere reibungsarm auf das Infusionsset zu übertragen. Vorstehende Aufgabe wird durch die Gegenstände des unabhängigen Anspruchs gelöst. Die abhängigen Ansprüche sind auf vorteilhafte Ausführungsformen gerichtet.

Die Insertionsvorrichtung gemäß der vorliegenden Erfindung wird vorzugsweise für medizinische Zwecke verwendet, insbesondere um Infusionssets in die Haut einzubringen, ohne dass der Patient direkt durch seine eigene Kraft das Infusionsset in die Haut einstechen muss. Diesen Einstechvorgang übernimmt für ihn die Insertionsvorrichtung. Die Insertionsvorrichtung weist vorzugsweise ein Gehäuse auf, das so gestaltet ist, dass es eine Nadel umgibt, die in dem Infusionsset enthalten ist, wenn das Infusionsset in der Insertionsvorrichtung angebracht ist. Weiter weist die Insertionsvorrichtung vorzugsweise eine Vortriebseinrichtung auf, um das Infusionsset zu bewegen, insbesondere zu beschleunigen, und zwar in eine vorbestimmte Richtung, die Vortriebsrichtung genannt wird. Die vorbestimmte Richtung kann insbesondere 90° zur Haut bzw. der Auflagefläche des Gehäuseunterteils sein. Sie kann aber auch für eine schräge Einstechrichtung ausgebildet sein, also ein Winkel zwischen 0 und 90°.

Die Insertionsvorrichtung gemäß der vorliegenden Erfindung ist so gestaltet, dass die Vortriebseinrichtung einen besonderen Aufbau aufweist, der so gestaltet ist, dass die Vortriebseinrichtung und insbesondere auch das Infusionsset gleitfrei, insbesondere reibungsfrei bezüglich des Gehäuses ist. Insbesondere sind also die beweglichen Teile der Vortriebsteile so gestaltet, dass sie nicht in Vortriebsrichtung gleiten, sondern bei einer Bewegung in Vortriebsrichtung gleitfrei und/oder kontaktfrei und/oder reibungsfrei sind. Insbesondere sind die sich in Vortriebsrichtung bewegenden Teile der Vortriebseinrichtung so gestaltet, dass sie von dem Gehäuse beabstandet sind.

Dies wird vorzugsweise dadurch erreicht, dass die Vortriebseinrichtung sich an dem Ende des Gehäuses (im folgenden auch als "oberes Ende" bezeichnet) abstützt, das dem Austrittsende des Infusionssets (im folgenden auch als "unteres Ende" bezeichnet) gegenüber liegt, wobei aufgrund der Konstruktion oder Beschaffenheit der Vortriebseinrichtung eine Bewegung in Richtung auf Seitenwände des Gehäuses, die entlang der Vortriebsrichtung verlaufen vorzugsweise vermieden oder vermindert wird, um einen Kontakt zwischen den sich in Vortriebsrichtung bewegenden Teilen der Vortriebseinrichtung und dem Gehäuse zu vermeiden.

Die Vortriebseinrichtung weist eine Vortriebsmechanik auf, die mit dem Gehäuse verbunden ist. Die Verbindung ist insbesondere an dem dem Austrittsende des Infusionssatzes entgegengesetzten Ende des Gehäuses (also am oberen Ende) gegeben. Sie kann aber auch, wie weiter unten ausgeführt wird, an einer Gehäuseseitenwand vorhanden sein. Im Folgenden wird als unteres Ende der Insertionsvorrichtung das Ende bezeichnet, aus dem das Infusionsset austritt, um in die Haut zu stechen. Das obere Ende ist das dazu entgegengesetzte Ende. Das obere und untere Ende wird durch seitliche Wände des Gehäuses verbunden.

Um die Vortriebseinrichtung und insbesondere die Vortriebsmechanik anzutreiben, wird zumindest ein elastisches Mittel verwendet, das durch Einwirkung von äußerer Kraft Energie speichern kann, wie zum Beispiel ein elastisches Mittel, das komprimiert wird. Jegliche andere mechanische, elektrische oder chemische Antriebsmittel können verwendet werden.

Die Insertionsvorrichtung gemäß der Erfindung weist ein Vortriebsendglied (z.B. Hammerkopf) auf, das ausgebildet ist, ein Infusionsset in Vortriebsrichtung zu bewegen. Das Vortriebsendglied steht zeitweise mit dem Infusionsset in Kontakt, um dieses zu beschleunigen.

Das Vortriebsendglied ist vorteilhaft relativ zum Gehäuse, insbesondere zum oberen Ende des Gehäuses und/oder zu einer Seitenwand des Gehäuses hin mittels eines elastischen Mittels und insbesondere unter zur Hilfenahme einer Vortriebsmechanik vorspannbar. Das Vortriebsendglied wird bei Freigeben der im Antriebsmittel gespeicherten Energie, also beim Lösen der Vorspannung in die Vortriebsrichtung bewegt. Dabei überträgt das Vortriebsendglied seinen Impuls auf das Infusionsset, das so gehaltert ist, dass es im Bewegungspfad des Vortriebsendglieds liegt, um so den Impuls des Vortriebsendglieds aufzunehmen.

Vorzugsweise ist die Vortriebseinrichtung in der vorgespannten Stellung verriegelbar, wobei vorzugsweise die Verriegelung durch Betätigen eines Freigabeglieds (zum Beispiel Schalter oder Druckknopf) entriegelbar ist.

Dabei weist die Vortriebseinrichtung eine Hebelmechanik auf. Diese dient insbesondere dazu, das Vortriebsendglied relativ zum Gehäuse zu bewegen, und zwar in Vortriebsrichtung. Die starren Glieder der Hebelmechanik, die sich um Drehpunkte drehen, werden im Folgenden als Schenkelhebel bezeichnet, während die Drehpunkte als Drehverbindungsglieder bezeichnet werden. Die Schenkelhebel der Hebelmechanik werden vorzugsweise zumindest zum Teil gegenüber dem Gehäuse vorgespannt, um dann eine durch die Anordnung der Schenkelhebel und Drehverbindungsglieder vorgegebene Bewegung so durchzufiihren, dass das Vortriebsendglied in Vortriebsrichtung bewegt wird. Die Hebelmechanik ist so gestaltet, dass die sich in Vortriebsrichtung bewegenden Teile in der Hebelmechanik vom Gehäuse beabstandet sind oder zumindest keine Gleitbewegung bezüglich des Gehäuses durchführen. Um dies zu erreichen und dennoch eine stabile Führung des Vortriebsendglieds in eine vorbestimmte Richtung zu erzielen, weist die Hebelmechanik vorzugsweise mindestens zwei Arme auf, die jeweils einen, zwei oder mehrere Schenkelhebel aufweisen, wobei die zwei oder mehreren Schenkelhebel eines Arms durch Drehverbindungsglieder verbunden sind. Diese beiden Arme sind vorzugsweise mechanisch so gekoppelt, dass sich eine Stabilisierung der Vortriebsbewegung dergestalt ergibt, dass einer Bewegung quer zur Vortriebsrichtung durch die Kopplung entgegengewirkt wird. Die Kopplung (z.B. durch Getriebe oder Zahnräder) ist vorzugsweise so gestaltet, dass sich die beiden Arme synchron in Vortriebsrichtung bewegen.

Die Hebelmechanik ist vorzugsweise so gestaltet, dass die Drehverbindungsglieder, über die die Schenkelhebel drehbar verbunden sind zumindest zum Teil in Vortriebsrichtung bewegbar sind, also insbesondere vom Gehäuse auch während einer Bewegung in Vortriebsrichtung beabstandet sind.

Insbesondere bei einer Gestaltung mit zwei Armen weist die Hebelmechanik zwei erste sich bei Bewegung in Vortriebsrichtung gegensinnig drehende Drehverbindungsglieder auf, die in Vortriebsrichtung bewegbar sind. Weiter sind vorzugsweise zwei zweite Drehverbindungsglieder vorgesehen, die aber ortsfest und insbesondere mit dem Gehäuse verbunden sind. Diese zwei zweiten Drehverbindungsglieder sind vorzugsweise in ihrer Drehbewegung gekoppelt, beispielsweise durch ein Getriebe, insbesondere Zähne. Auch ihre Drehbewegung ist vorzugsweise gegensinnig. Vorzugsweise befindet sich in einem Arm der Hebelmechanik ein erstes und ein zweites Drehverbindungsglied und in dem anderen Arm ebenso. In jedem Arm sind die ersten und zweiten Drehverbindungsglieder vorzugsweise über mindestens einen Schenkelhebel verbunden.

Gemäß einer weiteren bevorzugten Ausführungsform wird ein Vortriebsendglied über Hebel seitlich am Gehäuse angelenkt. Das Vortriebsendglied ist in diesem Fall vorzugsweise in Vortriebsrichtung länglich gestaltet, um zu ermöglichen, dass das Vortriebsendglied an zwei oder mehr Stellen mit der Gehäuseseitenwand über Hebel verbunden ist. Vorzugsweise ist am Vortriebsendglied mindestens ein Drehverbindungsglied vorgesehen, das mit mindestens einem Schenkelhebel verbunden ist. Vorzugsweise sind mindestens zwei Schenkelhebel zwischen dem Drehverbindungsglied am Vortriebsendglied und einem Drehverbindungsglied an der Gehäusewand vorgesehen. Vorzugsweise haben die beiden Schenkelhebel unterschiedliche Längen. Vorzugsweise sind die Längen so gestaltet, dass eine möglichst gradlinige Bewegung des Vortriebsendgliedes in Vortriebsrichtung ermöglicht wird. Dies wird insbesondere dadurch erreicht, dass zwischen den beiden Schenkelhebeln, die das Drehverbindungsendglied mit der Gehäusewand verbinden, ein weiteres Drehverbindungsglied vorgesehen ist, das nur über die Schenkelhebel mit dem Vortriebsendglied und der Gehäusewand verbunden ist. Vorzugsweise ist die Verbindung des Vortriebsendglieds mit der Gehäusewand in der Form einer Lemniskatenführung mit der Gehäusewand verbunden. Das Infusionsset wird mit dem unteren Ende des Vortriebsendglieds aus einer Halterung am Gehäuse geschlagen.

Um das Vortriebsendglied zu spannen, kann dieses über ein elastisches Mittel beispielsweise mit dem oberen Ende des Gehäuses verbunden sein. Alternativ oder zusätzlich können die Hebel gegen eine obere oder seitliche Wand des Gehäuses durch elastische Mittel vorspannbar gestaltet werden, um so das Vortriebsendglied nach unten zu beschleunigen, wenn sich das elastische Mittel entspannt. Außerdem kann natürlich wiederum ein Auslösemechanismus, ein Spanngriff und ein Rastmechanismus zum Einrasten des gespannten Zustands vorgesehen werden.

Bei einer weiteren erfindungsgemäßen Ausfiihrungsform ist das Vortriebsendglied als Schenkelhebel ausgebildet, wobei ein Ende des Schenkelhebels mit der Gehäusewand über ein Drehverbindungsglied verbunden ist. Es handelt sich dabei vorzugsweise um eine seitliche Gehäusewand. Das andere Ende dient dazu, ein Infusionsset aus einer Halterung zu schlagen. Ein elastisches Mittel spannt den Schenkelhebel gegen die Gehäusewand, so dass dieser weg von der Gehäusewand gedrängt wird, wenn die Spannung gelöst wird. Dabei schwenkt der Schenkelhebel vorzugsweise um das Drehverbindungsglied so, dass das freie Ende das Infusionsset aus der Halterung schlägt, wenn der Schenkelhebel senkrecht zur Applikationsrichtung des Infusionssets durch das elastische Mittel gespannt worden ist.

Gemäß einer weiteren Ausführungsform der Erfindung wird ein dehnbares elastisches Mittel vorgesehen, insbesondere ein Elastomerteil. Dieses ist so im Gehäuse angeordnet, dass es gedehnt wird, wenn das Vortriebsendglied entgegen der Applikationsrichtung bewegt wird. Dazu ist das Elastomerteil vorzugsweise unterhalb des Vortriebsendglieds verankert, gehaltert oder geführt und außerdem mit dem Vortriebsendglied verbunden. Da die Halterung, Führung oder Verankerung tiefer liegt als das Vortriebsendglied wird das Elastomerteil gespannt, wenn das Vortriebsendglied nach oben bewegt wird, bis es insbesondere eine Raststellung erreicht. Über einen Auslöser wird dann diese Raststellung gelöst, so dass das Vortriebsendglied mittels des gedehnten Elastomerteils, das sich wieder zusammenzieht, nach unten beschleunigt wird. Das Infusionsset kann wiederum durch das Vortriebsendglied aus einer Halterung geschlagen werden. Um eine möglichst geradlinige Bewegung nach unten parallel zu den Gehäuseinnenwänden zu erzielen, ist das Elastomerteil vorzugsweise symmetrisch um das Vortriebsendglied angeordnet und kann beispielsweise auf verschiedene Bänder aufgeteilt sein, die symmetrisch bezüglich des Bewegungspfades des Vortriebsendglieds angeordnet sind. Auch wie bei den anderen Ausführungsformen läuft der Bewegungspfad vorzugsweise parallel zur Gehäuseinnenwand. Insbesondere kann eine starre Hülse vorgesehen werden, um deren unteres Ende das Elastomerteil geführt wird, wobei das untere Ende der Hülse und damit des geführten Elastomerteils vorzugsweise zumindest in etwa mit der Austrittsebene übereinstimmt, aus der die Infusionsnadel bei der Applikation aus der Insertionsvorrichtung austritt. Das untere Ende des Elastomerteils ist insbesondere tiefer als das Vortriebsendglied, wenn sich dieses am oberen Ende seines Bewegungspfades befindet.

Das Infusionsset kann bei sämtlichen Ausführungsformen am Gehäuse gehaltert werden und zwar so, dass es in dem Bewegungspfad des Vortriebsendglieds hineinragt. Das Vortriebsendglied schlägt dann das Infusionsset aus der Halterung bei seiner Bewegung von oben nach unten und das Infusionsset fliegt im freien Flug auf die Haut zu, um die Nadel zu applizieren.

### Figurenbeschreibung:

Figur 1 ist eine schematische Darstellung einer ersten, nicht erfindungsgemäßen Insertionsvorrichtung.
Figur 2 ist eine modifizierte Ausführungsform der ersten, nicht erfindungsgemäßen Insertionsvorrichtung.
Figur 3 zeigt eine erste Stellung einer ersten erfindungsgemäßen Insertionsvorrichtung.
Figur 4 zeigt eine zweite Stellung der ersten erfindungsgemäßen Insertionsvorrichtung.
Figur 5 zeigt eine dritte Stellung der ersten erfindungsgemäßen Insertionsvorrichtung.
Figur 6 zeigt eine vierte Stellung der ersten erfindungsgemäßen Insertionsvorrichtung.
Figur 7 zeigt eine fünfte Stellung der ersten erfindungsgemäßen Insertionsvorrichtung.
Die Figuren 8a, 8b und 8c zeigen eine Variante der ersten erfindungsgemässen Ausführungsform.
Figur 9 zeigt die Variante der ersten erfindungsgemässen Ausführungsform im ausgelösten Zustand.
Figuren 10a, 10b und 10c zeigen eine zweite, nicht erfindungsgemässe Insertionsvorrichtung
Figur 11 zeigt eine zweite Ausführungsform der Erfindung.
Figuren 12a und 12b zeigen eine dritte, nicht erfindungsgemässe Ausführungsform der Erfindung.
Fig. 13a und 13b zeigen eine vierte, nicht erfindungsgemässe Insertionsvorrichtung.
Fig. 14a bis 14c zeigen weitere Ansichten der Insertionsvorrichtung der Fig. 13.
Fig. 15a bis 15c zeigen weitere Ansichten der Insertionsvorrichtung der Fig. 13.
Fig. 16 zeigt Bestandteile der Insertionsvorichtung der Fig. 13.

Bei der nachfolgenden Beschreibung bezeichnen gleiche Bezugszeichen, soweit nicht anders bemerkt, gleiche Teile. Verschiedene Merkmale verschiedener Ausführungsformen können miteinander kombiniert werden.

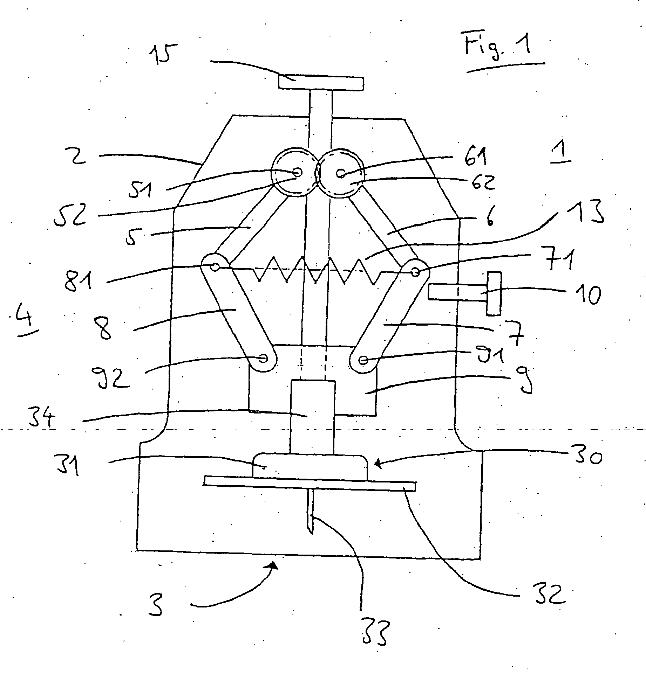

Fig. 1 zeigt eine schematische Darstellung einer nicht erfindungsgemässen Insertionsvorrichtung 1, mit einem Gehäuse 2, mit einer Gehäuseöffnung 3 an der Unterseite, einem im Gehäuse 2 axial verschiebbaren und lösbar gehaltenen Infusionsset 30 und einer entsprechenden Verschiebemechanik 4.

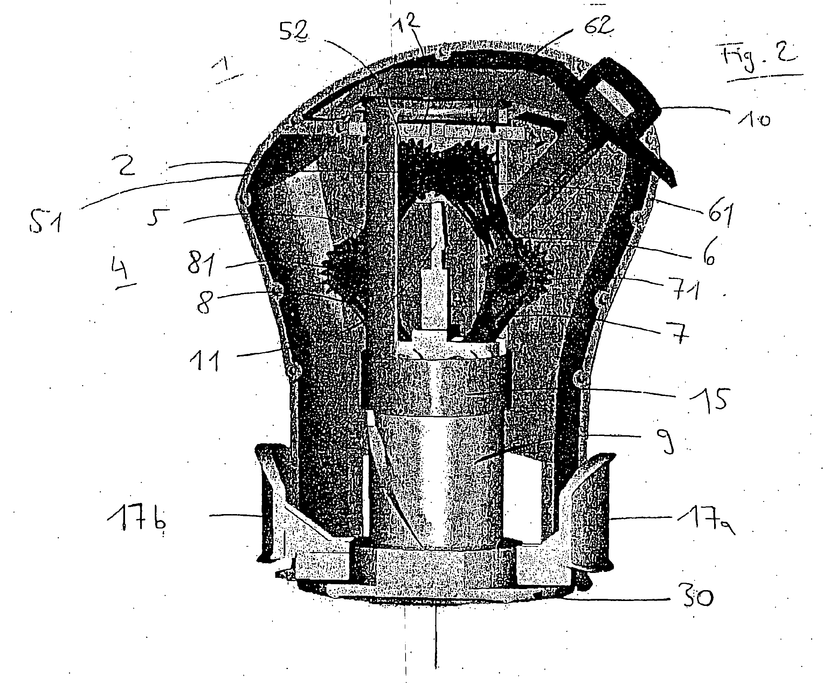

Die Verschiebemechanik 4 weist vier miteinander verbundene Schenkelhebel 5, 6,7, 8 auf. Die beiden oberen Schenkelhebel 5, 6 sind mittels eigener Lagerachsen 51, 61 an ihren Lagerenden 52, 62 im Gehäuse 2 drehgelagert. Zudem greifen die Lagerenden 52, 62 gezahnt (Fig. 2) ineinander - vergleichbar einem Zirkel, wie in DE 2357745 beschrieben. Beide oberen Schenkelhebel 5, 6 sind über Verbindungsachsen 71, 81 mit den unteren Schenkelhebeln 7, 8 verbunden, welche wiederum mittels Lagerachsen 91, 92 an einer Aufnahme 9 für das Infusionsset 30 gelagert sind. Durch diese Verbindung der einzelnen Schenkelhebel 5, 6, 7, 8 zueinander und zu Gehäuse 2 bzw. Aufnahme 9, hat ein Schwenken der oberen Schenkelhebel 5, 6 zueinander eine axiale Verschiebung der Aufnahme 9 und damit des Infusionssets 30 zur Gehäuseöffnung 3 zur Folge. Bei maximaler Verschwenkung der Schenkelhebel 5, 6 zueinander liegt die Unterseite 32 des Infusionssets etwas außerhalb der Gehäuseöffnung 3. Im weiteren verfügt die Verschiebemechanik über ein elastisches Element 13, z.B. eine Feder, welche in Fig. 1 zwischen den oberen Schenkelhebeln 5, 6 angeordnet ist und eine Schwenkbewegung der oberen Schenkelhebel 5, 6 zueinander bewirkt. Ebenfalls möglich wäre die Anordnung der Feder 13 zwischen Gehäuse 2 und mindestens einem Schenkelhebel 5, 6 vorzugsweise oberhalb der Verbindungsachsen 71 bzw. 81 zwischen oberen und unteren Schenkelhebeln.

Über eine Auslöseeinrichtung, beispielsweise einen Auslöseknopf 10, wird die Verschiebemechanik von einer gespannten in eine entspannte Position freigegeben.

Ein Infusionsset 30 verfügt üblicherweise über einen Kopf 31, ein daran angeordnetes Haltelement 34, eine klebende Unterseite 32 und eine die Haut durchdringende Kanüle 33. Je nach Ausgestaltung der Kanüle 33, aus leicht biegsamem Material z.B. Teflon, oder härterem Material, z.B. Stahl, kann die Kanüle 33 die Haut selbst penetrieren oder bedarf einer zusätzlichen Insertionskanüle (nicht angezeigt). Am Katheterkopf ist ein Infusionskatheter (nicht gezeigt) angeordnet, welcher eine Fluidverbindung zwischen der Kanüle 33 und einer Verabreichungsvorrichtung (nicht gezeigt) - z.B. einer Insulinpumpe - schafft.

Im Gebrauch wird ein Infusionsset 30, mittels Halteelement 34 an der Aufnahme 9 angeordnet und durch Bewegung des Aufnahmeelementes 9 weg von der Gehäuseöffnung 3, die Verschiebemechanik von der entspannten in die gespannte Position gebracht. Das Gehäuse 2 der Insertiönsvörcichtnng 1 wird mit der Gehäuseöffnung 3 auf die Haut gedrückt. Durch Betätigung des Auslöseknopfes 10 wird eine Bewegung der oberen Schenkelhebel 5, 6 zueinander und damit eine Bewegung der Aufnahme 9 und des Infusionssets 30 in Richtung Gehäuseöffnung 3 provoziert. Die Verschiebemechanik 4 drängt die Kanüle 33 unter die Haut und gleichzeitig die klebende Unterseite 32 an die Hautoberfläche. Um eine Loslösung des Katheterkopfes 31 von der Insertionsvorrichtung 1 möglichst schmerzfrei zu ermöglichen, wird durch Betätigung einer Auswurfeinrichtung 15 das Haltelement 34 von der Aufnahme 9 gelöst. Im einfachsten Fall ist das Haltelement 34 mittels Reibschluss in der Aufnahme 9 gehalten, so dass durch leichten axialen Druck über die Auswurfeinrichtung 15 - beispielsweise in Form einer Antriebsstange - auf das Halteelement 34 der Auswurf erfolgt.

Fig. 2 zeigt eine leicht modifiziert, nicht erfindungsgemässe Insertionsvorrichtung. Die Verschiebemechanik 4 weist auch hier vier verbundene Schenkelhebel 5, 6, 7, 8 auf, wobei die beiden oberen Schenkelhebel 5, 6 mittels Lagerachsen 51,61 an ihren Lagerenden 52, 62 im Gehäuse 2 gelagert sind und gezahnt ineinandergreifen. Beide oberen Schenkelhebel 5, 6 sind über Verbindungsachsen 71, 81 mit den unteren Schenkelhebeln 7, 8 verbunden, welche wiederum mittels Lagerachsen 91, 92 in einer Aufnahme 9 für das Infusionsset 30 gelagert sind. Die um diese Lagerachsen 91, 92 angeordneten Enden der unteren Schenkelhebel 7, 8 greifen in dieser Ausführungsform ebenfalls gezahnt ineinander (nicht gezeigt), um sicherzustellen, dass die Aufnahme 9 nicht seitlich einknicken kann. Ein solches Einknicken hätte zur Folge, dass die Kanüle 33 beim Insertionsvorgang nicht senkrecht zur Hautoberfläche bewegt würde.

Eine Betätigung des Auslöseknopfes 10 führt zu einer Seitwärtsbewegung einer an der Aufnahme 9 angeordneten Rastnase 11 aus einer im Gehäuse 2 angeordneten Halterung 12 und damit zu einer Freigabe der Verschiebemechanik 4 von einer gespannten in eine entspannte Position.

Im Gebrauch wird durch Bewegung von Greifelementen 17a, 17b in Richtung Auslöseknopf 10 die Verschiebemechanik 4 gespannt und die Rastnase 11 in die Halterung 12 eingerastet. Eine Betätigung des Auslöseknopfes 10 führt zu einer Seitwärtsbewegung der Rastnase 11-und damit zu einer Lösung aus der Halterung 12 und damit zu einer Freigabe der Verschiebemechanik 4 von einer gespannten in eine entspannte Position. Ist die Kanüle 33 unter der Haut angeordnet erfolgt durch erneute Betätigung des Auslöseknopfes 10 eine leichte axiale Bewegung der Auswurfeinrichtung 15 und damit über (nicht gezeigte) Nocken eine axiale Bewegung auf das mittels Reibschluss in der Aufnahme 9 gehaltene Infusionsset 30. Diese Bewegung genügt, um das Infusionsset 30 aus der Aufnahme 9 auszuwerfen.

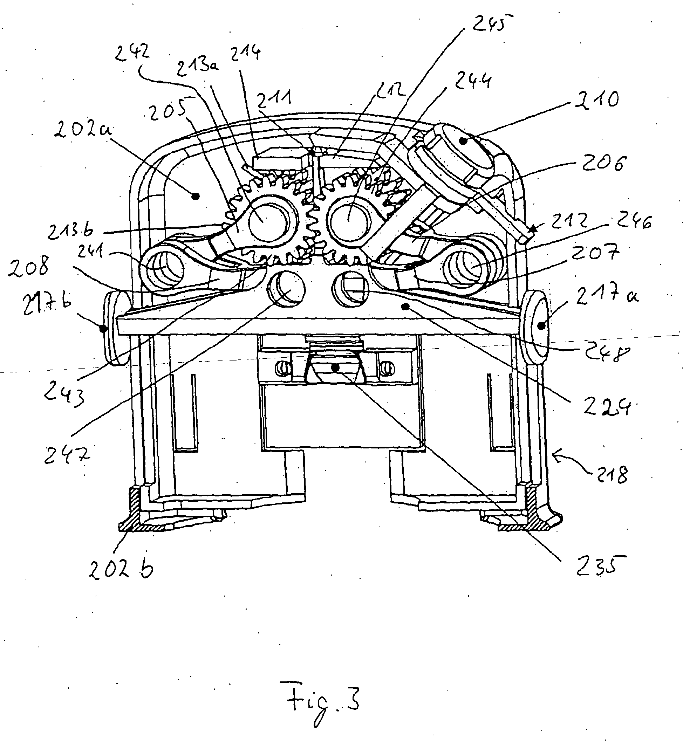

Die Figuren 3 bis 7 zeigen eine erfindungsgemäße Insertionsvorrichtung, die ebenfalls auf dem Prinzip einer Hebelmechanik beruht. Fig. 3 zeigt die Insertionsvorrichtung ohne lnfusionsset im gespannten Zustand. In Fig. 3 ist eine Feder 213 eingezeichnet, die an den Enden 213a, 213b sichtbar sind. Ein Ende 213a stützt sich an einem Widerlager 214 ab, das ortsfest mit dem Gehäuse 202a verbunden ist. Das andere Ende der Schenkelfeder 213 drückt gegen den Schenkelhebel 205 und versucht diesen in Fig. 3 nach unten zu drücken.

Daran wird die Feder durch den Riegel 211 gehindert, der mit einem Haken an einer Rippe 212 am oberen Ende des Gehäuses verhakt ist. Die Rippe 212 ist ortsfest mit dem Gehäuse 202 verbunden. Der Schenkelhebel 205 ist mit einem anderen Schenkelhebel 208, der darunter angeordnet ist, über ein Drehverbindungsglied (Achse) 241 verbunden, das eine Drehung der Schenkelhebel 205 und 208 um die gemeinsame Achse 241 gewährleistet. Ein weiteres Drehverbindungsglied 242 ist am oberen Ende des Schenkelhebels 205 angeordnet. Die Drehachse des Verbindungsgliedes 242 ist im Gegensatz zur Drehachse 241 ortsfest. Auf der Achse des Verbindungsgliedes 242 ist ein Zahnrad 243 angeordnet, das sich mit dem oberen Ende des Schenkelhebels 205 mitdreht. Das Zahnrad 243 kämmt mit einem Zahnrad 244, das sich um ein Drehverbindungsglied 245 bzw. eine Achse 245 dreht. Dieses Zahnrad 244 ist mit dem oberen Ende des Schenkelhebels 206 so verbunden, dass es sich mit diesem mitdreht. So bewirkt eine Drehbewegung des Schenkelhebels 205 um die Achse 242 über die kämmenden Zahnräder eine entsprechende gegensinnige Drehbewegung des Schenkelhebels 206 um die Achse 245. Vorzugsweise ist eine zweite Schenkelfeder vorgesehen, deren einer Schenkel sich ebenfalls am Gehäuse abstützt, während der andere den Schenkelhebel 206 nach unten drängt. Eine dadurch bewirkte Rotationsbewegung um die Achse 245 wird ebenfalls über die Zahnräder 244 und 243 auf den Schenkelhebel 205 übertragen.

Der Schenkelhebel 205 ist über das Drehverbindungsglied 241 mit dem Schenkelhebel 208 verbunden, der wiederum über ein Drehverbindungsglied 247 mit einem Hammerkopf oder Kolben 224 verbunden ist, der als Vortriebsendglied dient. Entsprechend ist der Schenkelhebel 207 über ein Drehverbindungsglied 248 mit dem Hanunerkopf 224 verbunden.

Greifelemente 217a und 217b sind fest mit dem Hammerkopf 224 verbunden. Der Hammerkopf 224 steht durch beidseitig des Gehäuses befindliche Längsschlitze 218 hindurch, und zwar vorzugsweise berührungslos, um keine Reibungsverluste zu haben. In der in Fig. 3 gezeigten Stellung ist der Hammerkopf an seinem obersten Ende.

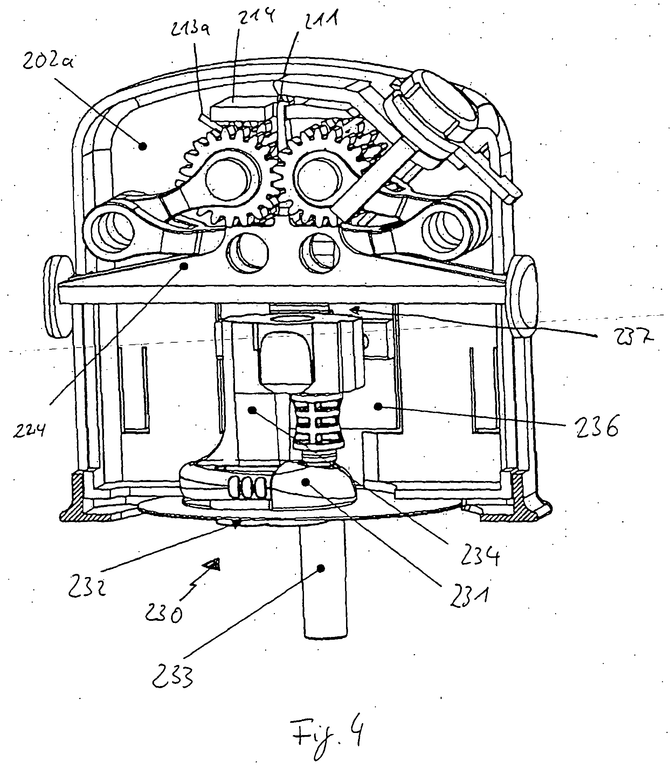

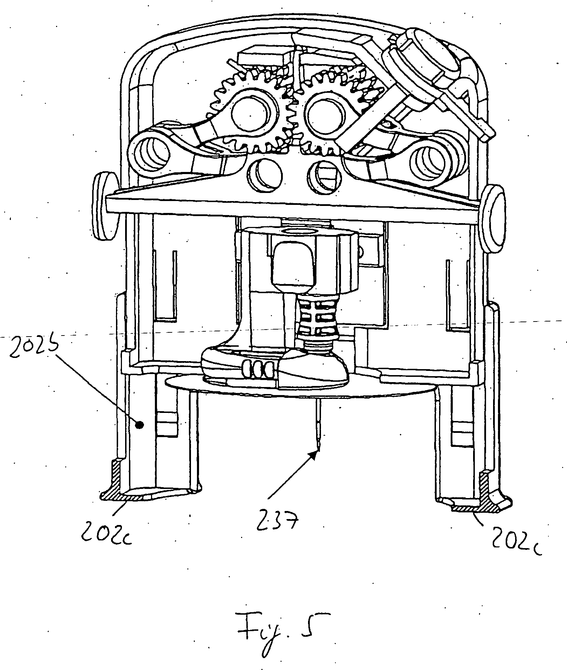

Eine Aufnehmung oder Aufnahme 235 dient dazu, ein Infusionsset aufzunehmen, wie dies in Fig. 4 zu sehen ist. Das Gehäuse ist vorzugsweise zweiteilig ausgebildet, mit den Teilen 202a und 202b, wobei das Teil 202b nach unten geschoben werden kann, wie später im Zusammenhang mit Fig. 5 diskutiert wird.

Wie bereits erwähnt, ist ein Infusionsset 230 mit den Bestandteilen Kopf 231, Hauptteil 234, Schutzhülse für die Kanüle 233 und einem sogenannten Liner bzw. ein Klebepflaster 232 gezeigt. Das Infusionsset 232 ist an der nunmehr in Fig. 4 verdeckten Aufnahme 235 angebracht, die mit der Hinterwand 236 des Gehäuses verbunden ist. Wie in Fig. 4 zu sehen ist, befindet sich ein Spalt 237 zwischen dem Infusionsset 230 und dem Hammerkopf 234, wenn sich dieser im eingerasteten Zustand befindet, das heißt die Rastnase oder der Riegel 211 ist eingerastet, wie dies bereits im Zusammenhang mit Fig. 3 diskutiert wurde.

Fig. 5 zeigt nun die Insertionsvorrichtung im ausführbereiten Zustand. Die Insertionsvorrichtung ist mit dem Infusionsset geladen. Die Schutzhülse 233 wurde entfernt, so dass nun die Kanüle 237 frei vorsteht. Die untere Gehäusehälfte 202b ist ausgefahren und umgibt somit schützend die freiliegende Kanüle 237. Die unteren Enden 202c des Gehäuses 202b sind vorzugsweise flach ausgebildet, um eine gute Auflage des Gehäuses auf der Haut zu gewährleisten.

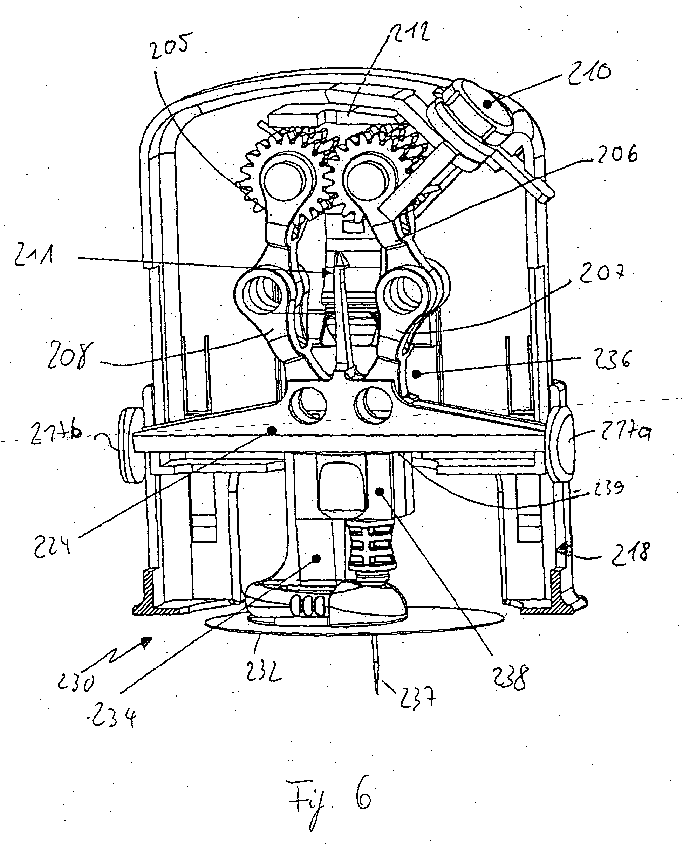

Fig. 6 zeigt die Insertionsvorrichtung nachdem der Auslöseknopf 210 betätigt wurde und die Kanüle in die Haut eingedrungen ist. Wie man sieht steht der Liner 232 unterhalb des Gehäuses hervor und insbesondere natürlich die Kanüle oder Nadel 237, so dass diese ungehindert vom Gehäuse in die Haut eindringen kann. Die Schenkelhebel 205 und 206 wurden durch die sich entspannende Schenkelfeder 213 und eine weitere nicht gezeigte Schenkelfeder nach unten gedrängt, nachdem die Rastnase 211 durch den Auslöseknopf 210 von der Rippe 212 gelöst worden ist. Die Schenkelhebel 205, 206, 207 und 208 sind nun im gestreckten Zustand und haben sich um die jeweiligen Verbindungsglieder gedreht. Dadurch wurde der Hammerkopf 224 nach unten in seine Vortriebsrichtung getrieben. Aufgrund des ausreichend breit bemessenen Spalts 218 ist der Hammerkopf auch gleitfrei und kontaktlos in diesem Spalt vorwärts bewegt worden und die Greifelemente 217a und 217b befinden sich nun in ihrem unteren Endzustand.

Während der Hammerkopf nach unten vorwärtsgetrieben wurde, hat er gegen das obere Ende 239 des Infusionssets 230 geschlagen und es so aus der Aufnehmung 235 herausgelöst, in der das Infusionsset durch Form, Kraft und/oder Reibschluss gehalten wurde. Aufgrund der Trägheit fliegt von da an das Infusionsset 230 in dieselbe Richtung, wie sich der Hammerkopf 224 bewegt und sticht die Kanüle 237 in die Haut ein. Das Infusionsset bewegt sich also gemäß einem freien Flug und ungeführt. Dennoch konnten optimale Einstichergebnisse erzielt werden, wobei insbesondere Reibungsverluste aufgrund einer Führung des Infusionssets vermieden wurden.

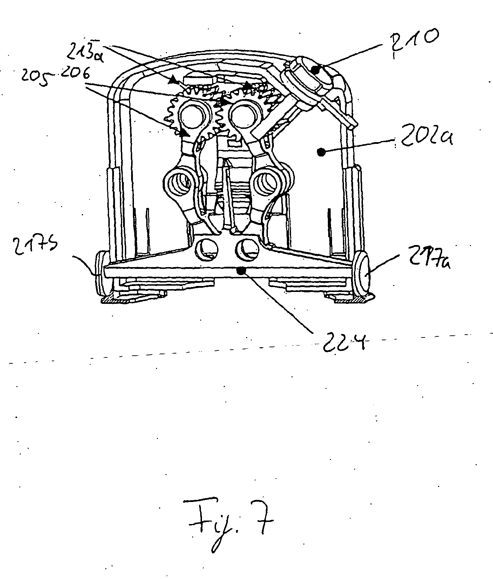

Fig. 7 zeigt nun die Insertervorrichtung in einem Zustand bevor sie wieder erneut gespannt wird und in einem Zustand in dem noch kein Infusionsset eingebracht worden ist. Durch Hochdrücken der Greifelemente 217a und 217b gegen die Feder 213 wird der Hammerkopf 224 wieder nach oben in seine geladene Stellung gebracht.

Die Halterung des Infusionssets am Gehäuse kann verschieden gestaltet werden. Beispielsweise kann das obere Ende des Infusionssets zwischen zwei Aufnahmen 235, die als Klemmhalterungen dienen kraftschlüssig gehalten werden. Dabei ist eine Klemmhalterung 235 an einer Wand des Gehäuses 202a angebracht und die andere ist gegenüberliegend. Der Abstand zwischen den Klemmhalterungen ist größer als die Breite des Hammerkopfes 224, so dass dieser kontaktfrei zwischen zwei gegenüberliegenden Klemmhalterungen 235 hindurchgelangen kann und dabei das Infusionsset mitnehmen kann und aus der Klemmhalterung lösen kann.

Alternativ zur Klemmung kann auch die Aufnahme 235 so gestaltet werden, dass sie beispielsweise Fortsätze aufweist, die nach unten weisen und das Infusionsset wird dann von unten nach oben auf diese Fortsätze aufgesteckt, wobei das Infusionsset entsprechende Vertiefungen aufweist, um beispielsweise mittels Reibschluss die Steckverbindung zwischen Infusionsset und Fortsatz herzustellen. Der Fortsatz und die Vertiefung ist dabei wiederum vorzugsweise so angeordnet, dass der Hammerkopf bei seiner Bewegung in Vortriebsrichtung kontaktfrei daran vorbeigelangen kann und dann das Infusionsset mitreißt bzw. freischlägt.

Ergänzend zur obigen Beschreibung wird noch bemerkt, dass die Schenkelfedern um die Achsen 242 und 245 vorzugsweise gelagert sind. Die Aufnahmen 235 sind für den Fall einer reibschlüssigen oder kraftschlüssigen Verbindung mit dem einzubringenden Infusionsset vorzugsweise mit einem elastischen Mittel versehen, das insbesondere senkrecht oder quer zur Vortriebsrichtung wirkt, wobei vorzugsweise zwei einander gegenüberliegende elastische Mittel das Infusionsset klemmend festhalten, wobei dies natürlich auch durch ein einziges bewerkstelligt werden kann.

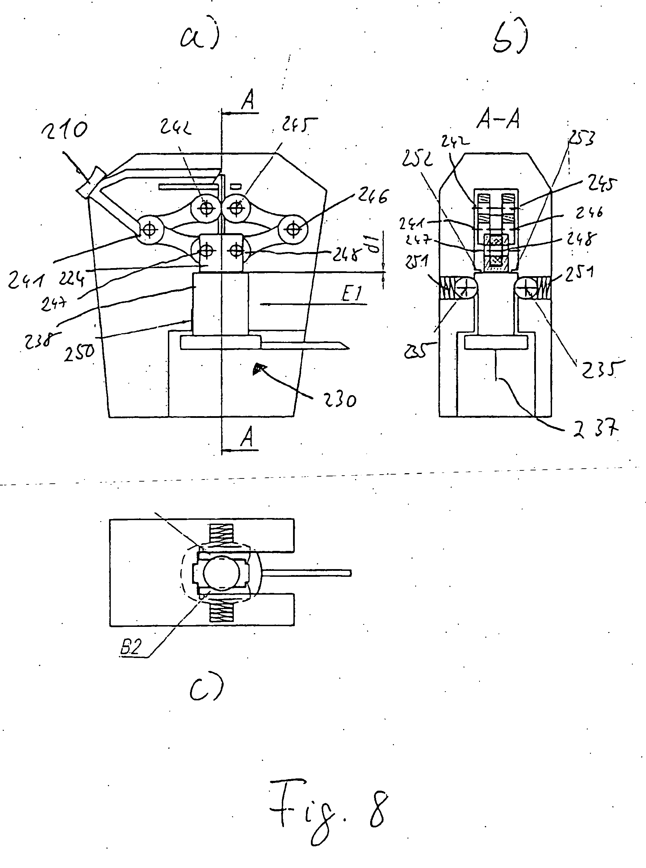

Wie aus Fig. 8b ersichtlich, drücken die Aufnehmer 235 seitlich gegen das Infusionsset, um dieses festzuklemmen. Das Drücken erfolgt mittels der elastischen Elemente 251, bei denen es sich beispielsweise um Spiralfedern handelt. Fig. 8c zeigt eine Draufsicht auf die Fig. 8b. Schlägt nun der Hammerkopf 224 auf das obere Ende des Infusionssets 230, so werden die Aufnehmer 235 zurückgedrängt gegen die Federkraft der Federn 251 und das Infusionsset fliegt frei beschleunigt durch die Kraft des aufschlagenden Hammers nach unten. Fig. 8b ist eine Schnittansicht entlang der Linie A-A der Fig. 8a.

Vorzugsweise sind die Aufnehmer 235 als Druckbolzen ausgebildet, die beim Einführen des Infusionssets ein hörbares Einklicken bewirken, um dem Patienten anzuzeigen, dass das Infüsionsset richtig eingefügt wurde. Vorzugsweise sind noch oberhalb des Infusionssets Anschläge 252 und 253 angebracht, um eine korrekte Lage des Infusionssets zu erzielen. Der Abstand d1 zwischen Hammerkopf und Infusionsset liegt vorzugsweise im Bereich bis 10 mm, d.h. kleiner als 10 mm.

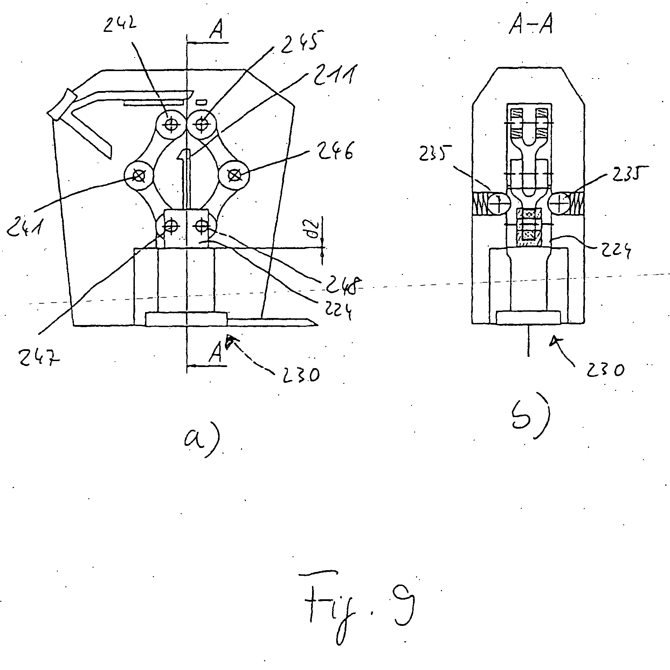

Vorzugsweise wird erst nach dem Festklemmen das Infusionsset in seiner Ausgangslage, also in der gespannten, in Fig. 8 gezeigten Stellung, die Schutzhülse (Nadelschutz) und insbesondere das Schutzpapier entfernt. Aufgrund des Festklemmen des Infusionssets und Verriegelung mittels der Rastnase 211 kann dies gefahrlos erfolgen. Die Insertionsvorrichtung ist nun bereit, das Infusionsset zu applizieren. Hierzu wird das Infusionsset auf die Haut an der vorgesehenen Stelle aufgesetzt. Zum Auslösen muss nur der Knopf 210 betätigt werden. Dadurch wird die Rastnase (Schnapphaken) ausgelenkt und die Schnappverbindung somit gelöst. Die vorgespannte Mechanik wird dann nach unten beschleunigt und der Hammerkopf schlägt nach einem Freiflug (ohne Kontakt mit dem Gehäuse) auf das Infusionsset auf. Der Hammerkopf übt auf das Infusionsset eine vertikale Kraftkomponente nach unten aus, welche sich seitlich auf die Aufnehmer 235 (Druckbolzen) überträgt. Dadurch werden die Federn zusammengedrückt, was zu einer Freigabe des Infusionssets führt. Das Infusionsset wird nun durch die Federkraft der vorgespannten Hebel in den Körper appliziert. Der in Fig. 9a gezeigte Abstand d2 zwischen Hammerkopf und Infusionsset nach dem Auslösen muss nicht definiert sein. Je nach Wölbung der Haut an der Applikationsstelle ist das Infusionsset mit dem Hammer in Kontakt oder nicht. Nach dem Einstechvorgang kann nun die Insertionsvorrichtung von der Applikationsstelle entfernt werden und der Applikationsvorgang ist abgeschlossen.

Bei einer weiteren, in den Figuren nicht gezeigten und nicht erfindungsgemässen Variante wird das Infusionsset nicht am Gehäuse befestigt, um dann durch den Hammerkopf davon losgeschlagen zu werden, sondern das Infusionsset wird an dem Hammerkopf selbst angebracht. Das heißt, insbesondere das obere Ende des Infusionssets wird mit dem unteren Ende des Hammerkopfs in Verbindung gebracht. Dies kann z.B. durch Kleben, Reibschluss oder Formschluss erfolgen. Insbesondere kann das Infusionsset auf den Hammerkopf aufgesteckt werden. Dabei kann beispielsweise der Hammerkopf einen Fortsatz aufweisen, der in eine entsprechende Kammer passende Ausnehmung im Infusionsset beispielsweise passgenau eingreift, um das Infusionsset reibschlüssig zu halten. Natürlich kann dies auch umgekehrt ausgebildet sein, das heißt das Infusionsset hat einen Fortsatz und der Hammerkopf eine entsprechende Ausnehmung.

Auch kann bei einer nicht erfindungsgemässen Variante das Infusionsset den Hammerkopf klemmend umgreifen, um daran befestigt zu werden oder umgekehrt. Besonders vorzugsweise wird dabei darauf geachtet, dass bei der Bewegung in Vortriebsrichtung weder das Infusionsset noch der Hammerkopf mit dem Gehäuse in Berührung kommen, um so unnötige Reibungsverluste bei der Umsetzung der potentiellen Energie des Antriebsmittels in die kinetische Energie des Infusionssets zu vermeiden. Dies erlaubt es die Insertionsvorrichtung kompakt ohne übermäßig große Antriebsmittel zu gestalten.

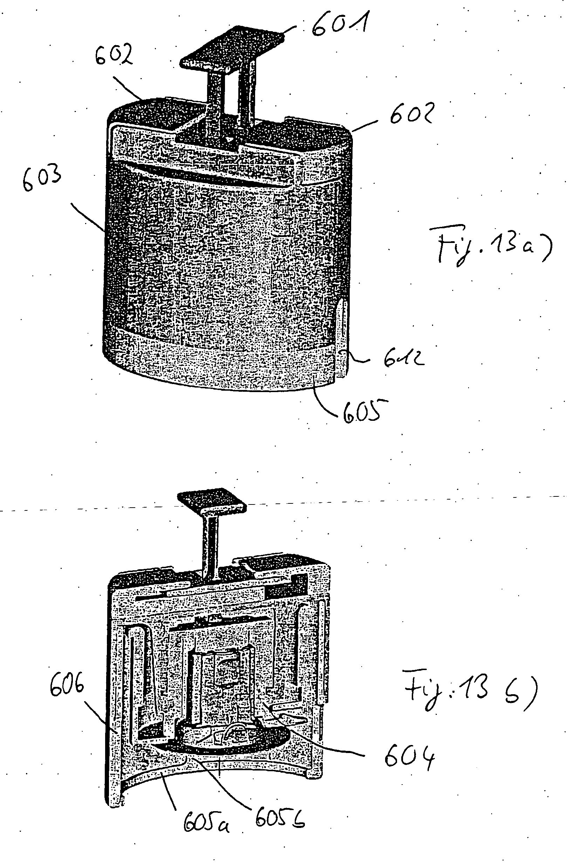

Fig. 9 verdeutlicht nochmals das Prinzip der vorliegenden Erfindung, wobei gleiche Bezugszeichen gleiche Teile wie bei der zuvor beschriebenen Ausführungsform bezeichnen. Die bezüglich Fig. 8 und 9 beschriebene Variante der ersten erfindungsgemässen Ausführungsform ähnelt dieser. Anhand der Fig. 9 wird eine seitliche Einführung des Infusionssets in den Inserter bzw. die Insertionsvorrichtung beschrieben. Das Insertionsset 230 wird seitlich in die Richtung E1 in die Insertionsvorrichtung eingeschoben. Der Abstand zwischen dem Hammerkopf 224 und dem oberen Ende 238 des Infusionssets ist mit "d1" bezeichnet. Dieser Abstand kann wie bei der vorherigen Ausführungsform sein. Eine Führung 250 dient als Anlagefläche für das Infusionsset, um dieses passend gegenüber dem Hammer 224 auszurichten. Entsprechend kann auch bei der ersten erfindungsgemässen Ausführungsform die in Fig. 4 gezeigte hintere Wand 236 gestaltet sein, um das Infusionsset zu führen, wenn es in die Insertionsvorrichtung eingebracht wird. Die seitliche Einführung des Infusionssets kann auch bei den anderen Ausführungsformen vorgesehen werden. Beispielsweise ist bei der in den Figuren 13 bis 16 gezeigten, nicht erfindungsgemässen Insertionsvorrichtung hierzu ebenfalls seitlich ein Spalt 612 (Fig. 13a) vorgesehen.

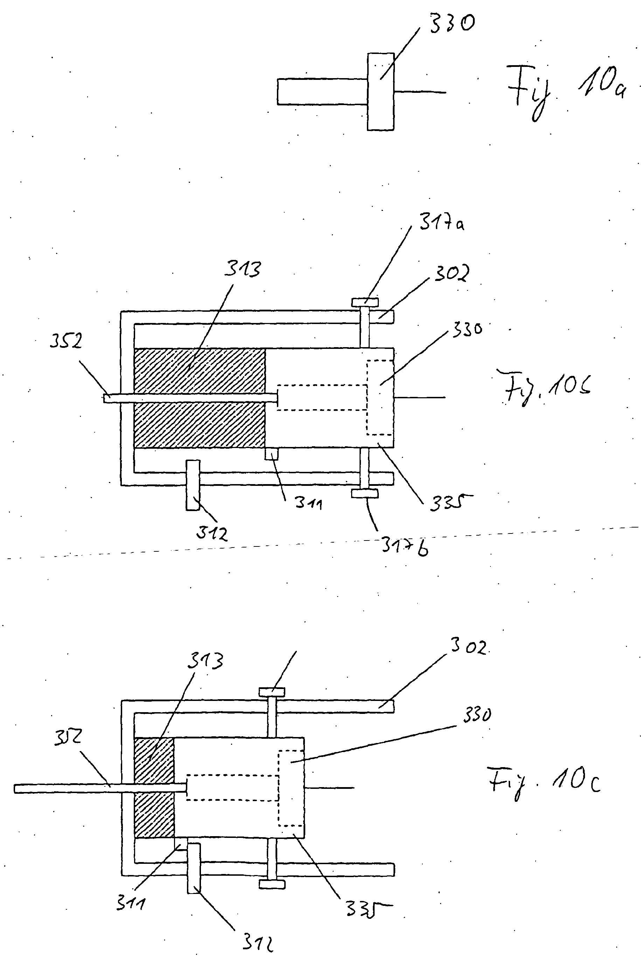

Fig. 10 zeigt eine zweite, nicht erfindungsgemässe Insertionsvorrichtung, bei der ein elastisches Mittel bevorzugt sowohl als Führung für das Vortriebsendglied als auch als Antrieb dient. Bei der in Fig. 10 gezeigten Ausführungsform ist das elastische Mittel aus einem elastischen Material gebildet und nimmt vorzugsweise eine längliche Form mit in Längsrichtung gleichbleibendem Querschnitt ein, um so im entspannten Zustand einen über die gesamte Längsrichtung gleichbleibenden Abstand zum Gehäuse zu haben. Das elastische Mittel ist vorzugsweise aus einem elastischen Material gebildet, wie zum Beispiel Schaumstoff oder Gummi. Fig. 10a zeigt das Infusionsset, das in Fig. 10b in die Aufnahme 335 eingefügt ist. Die Aufnahme dient zum Halten des Infusionssets beispielsweise durch Reib- oder Kraftschluss. Die Griffe 317a und 317b verlaufen in einem Längsschlitz des Gehäuses und dienen dem Laden der Insertionsvorrichtung, indem das elastische Mittel 313 von einem entspannten Zustand (Fig. 10b) in einen gespannten Zustand (Fig. 10c) gebracht wird. Dabei rastet der Nocken 311 in den Auslöser 312 ein. Der Auswerfer 352 wird bei dieser Bewegung mitgenommen. Wird nun in Fig. 10c der Auslöser 312 von dem Nocken 311 weg bewegt, so entlädt sich die im elastischen Mittel 313 gespeicherte Energie und treibt das Vortriebsendglied 335, das gleichzeitig als Aufnahme ausgebildet ist, in die Vortriebsrichtung.

Nachdem so das Infusionsset auf der Haut appliziert wurde, dient der Auswerfer 352 zum Lösen des Infusionssets aus der Aufnahme 335.

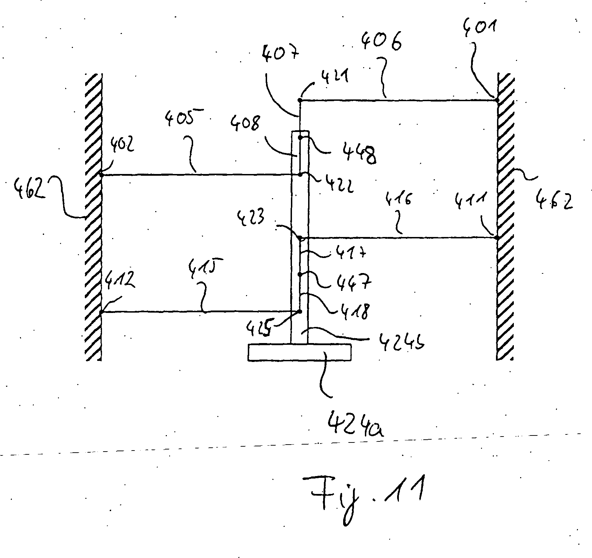

Fig. 11 zeigt eine zweite Ausführungsform der Erfindung, bei der ein Vortriebsendglied in der Form eines Stempels oder umgedrehten T-s gestaltet ist. Das Vortriebsendglied weist ein unteres Ende 424a auf, an das ein Infusionsset anbringbar ist oder das ausgebildet ist, dieses aus einer Halterung zu schlagen, die beispielsweise an einer Gehäusewand befestigt ist. Weiter weist das Vortriebsendglied vorzugsweise ein in Vortriebsrichtung längliches Teil 424b auf, das hier als Verbindungsstange bezeichnet wird. An dieser Verbindungsstange sind Drehlager 448 und 447 angebracht, die als Drehverbindungsglieder zu Hebeln 408 und 407 sowie 417 und 418 jeweils dienen. Die Drehverbindungsglieder 421, 422, 423 und 425 sind nicht mit der Verbindungsstange 424 fest verbunden, sondern nur über die Hebel 407, 408, 417 und 418 jeweilig. Diese Drehverbindungsgfieder sind wiederum jeweilig über Schenkelhebel 405, 406, 415 und 416 mit Drehverbindungsgliedem bzw. Drehlagern 401, 402, 411, 412 an der Gehäusewand verbunden. Die gezeigte Ausführungsform erlaubt eine Führung des Verbindungsendgliedes gemäß einer Lemniskaten-Kurve und wird deshalb auch als Lemniskatenführung bezeichnet. Die Verbindungsstange wird dadurch zumindest im Wesentlichen vertikal geführt. Elastische Spannmittel sowie der Einrastmechanismus und Auslösemechanismus können analog zu den anderweitig beschriebenen Ausführungsformen ausgebildet sein. Insbesondere können die elastischen Mittel mit der Verbindungsstange und/oder mit einem oder mehreren der Schenkel hebel verbunden sein, um diese so vorzuspannen, dass das Verbindungsendglied nach unten beim Entspannen der elastischen Mittel beschleunigt wird.

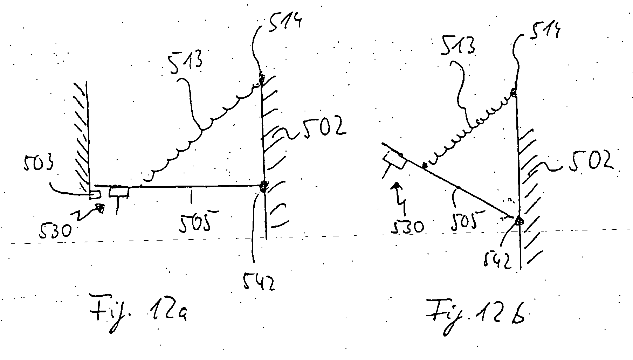

Fig. 12 zeigt eine dritte, nicht erfindungsgemässe Ausführungsform der Erfindung. An der Gehäusewand 502 ist über ein Drehverbindungsglied 542 ein Schenkelhebel 505 mit einem Ende angebracht. An dem gegenüberliegenden Ende des Schenkelhebels ist ein Infusionsset 530 befestigt. Über eine Feder 513 ist der Schenkelhebel gegen die Gehäusewand 502 spannbar, wie dies in Fig. 12b gezeigt ist. Wird ein nicht gezeigter Rastmechanismus gelöst, so wird durch die Feder 513 der Schenkelhebel 505 nach unten gedrängt, wie dies in Fig. 12a gezeigt ist. Schlägt ein freies Ende des Schenkelhebels gegen einen Anschlag 503, so kann sich das Infusionsset 530 zum Beispiel aus einer reibschlüssigen Verbindung lösen und nach einem kurzen freien Flug in die Haut eindringen.

Anstelle des Anschlags kann natürlich auch ein Auswurfmechanismus vorgesehen sein, mit dessen Hilfe das Infusionsset nach Eindringen in die Haut von der Insertionsvorrichtung gelöst werden kann. Alternativ kann das Infusionsset auch durch den Schenkelhebel 505 aus einer Halterung geschlagen werden, wie dies bei anderen Ausführungsformen beschrieben wurde. Letztgenannte Ausführungsform ist erfindungsgemäss.

Fig. 13 bis 16 zeigen eine vierte, nicht erfindungsgemässe Insertionsvorrichtung. Fig. 13a zeigt die Insertionsvorrichtung im gespannten Zustand, das heißt der Ladegriff 601 ist nach oben gezogen. Links und rechts vom Ladegriff befindet sich der Auslöser 602. Zum Auslösen werden diese nach innen gedrückt. Das Gehäuse ist mit 603 bezeichnet und umgibt ein Vortriebsendglied 604, das in Fig. 13b zu sehen ist. Am unteren Ende des Gehäuses 603, mit diesem vorzugsweise einstückig verbunden, befindet sich ein Elastomerteil 605. Dieses ist um eine Hülse 606 am unteren Ende der Insertionsvorrichtung herum geführt, wie aus Fig. 13b ersichtlich ist. Das Elastomerteil liegt an der unteren Innenwand der Hülse 606 an einer mit 605a bezeichneten Stelle an. Von dort aus verzweigt es in Form eines Bandes 605b nach oben. Fig. 13b zeigt ebenfalls den gespannten Zustand.

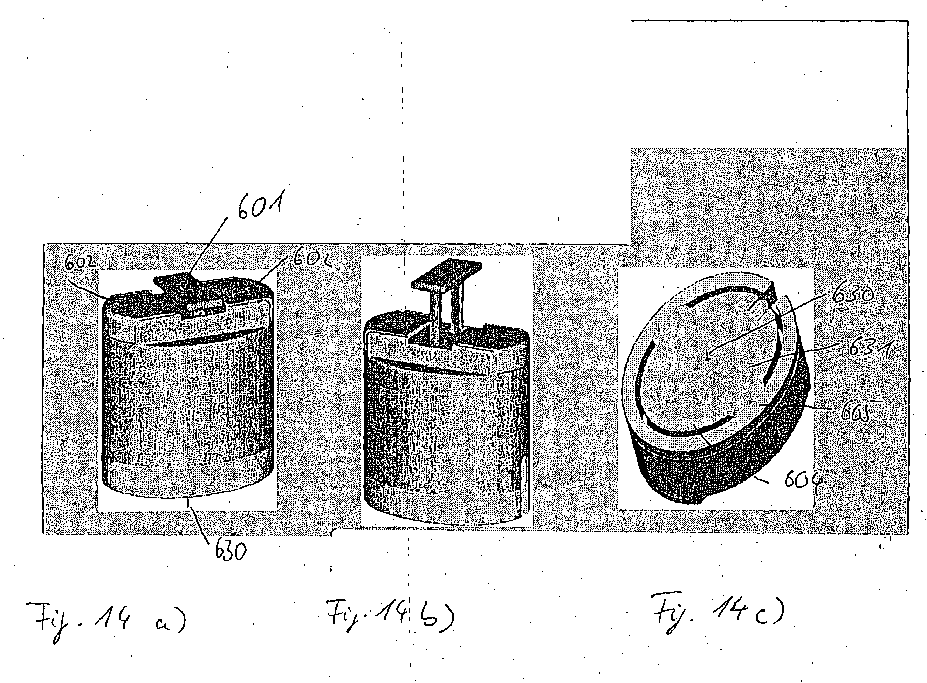

Fig. 14a zeigt den entspannten Zustand, in dem der Ladegriff 601 sich nach unten bewegt hat, nachdem die Auslöser 602 betätigt worden sind. Eine Nadelspitze 630 ist unten erkennbar, die aus der Insertionsvorrichtung unten herausragt. Fig. 14b entspricht Fig. 13a. Fig. 14c zeigt den Zustand der Fig. 14a mit der Infusionsnadel 630 und dem Liner bzw. Pflaster 631. Das Vortriebsendglied 604 befindet sich an dem unteren Ende seines Bewegungspfades. Das unterste Ende des Vortriebsendglieds 604 liegt zumindest in etwa in der Ebene des unteren Endes des Elastomerteils 605. Wie aus Fig. 14c ersichtlich besteht ein Abstand zwischen dem unteren Ende 605 des Elastomerteils und dem Vortriebsendglied 604.

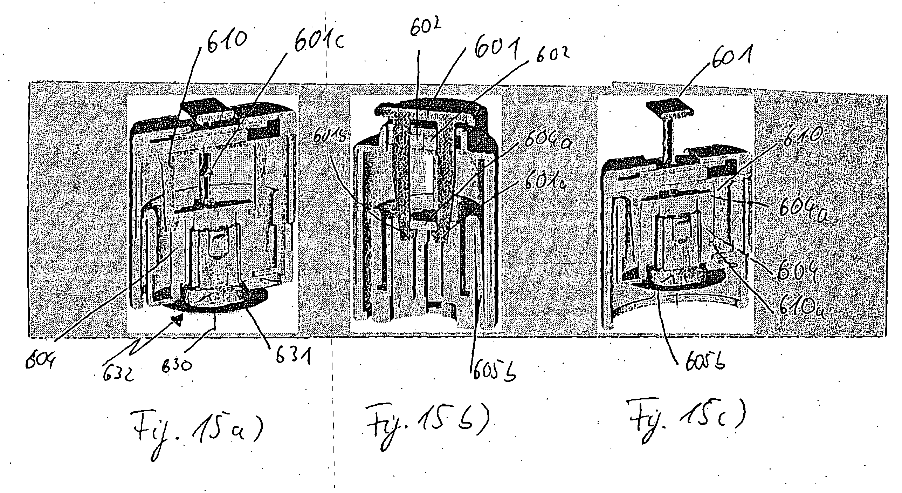

Fig. 15a zeigt im Schnitt den entspannten Zustand der Fig. 14a. Das Infusionsset 632 ist in dem Vortriebsendglied 604 gehaltert. Haken 610 sind mit dem Vortriebsendglied 604 verhakt und definieren die tiefste Stellung des Bewegungspfades des Vortriebsendglieds.

Wie aus Fig. 15b ersichtlich ist, ist das Band 605b des Elastomerteils um ein oberes Ende des Vortriebsendglieds geführt, so dass das Band gedehnt wird, wenn das Vortriebsendglied nach oben bewegt wird. Die Bewegung nach oben wird mit Hilfe des Ladegriffs 601 bewirkt. Dieser Ladegriff weist an seinem unteren Ende zwei Haken 601a und 601b auf, die mit einem entsprechend gestalteten Vorsprung 604a des Vortriebendglieds verrastet sind. Diese Verrastung lässt sich durch die Auslöser 602 lösen, indem diese im herausgezogenen Zustand des Ladegriffs 601 auf in Fig. 15a zu sehende Fortsätze 601 c wirken.

Fig. 15c zeigt den gespannten Zustand im Schnitt. Das Band 605b ist gespannt und die Insertionsvorrichtung ist bereit zur Applikation, wobei das Vortriebsendglied 604 sich nach unten bewegt, bis Fortsätze 604a gegen die hakenförmigen Enden 610a schlagen.

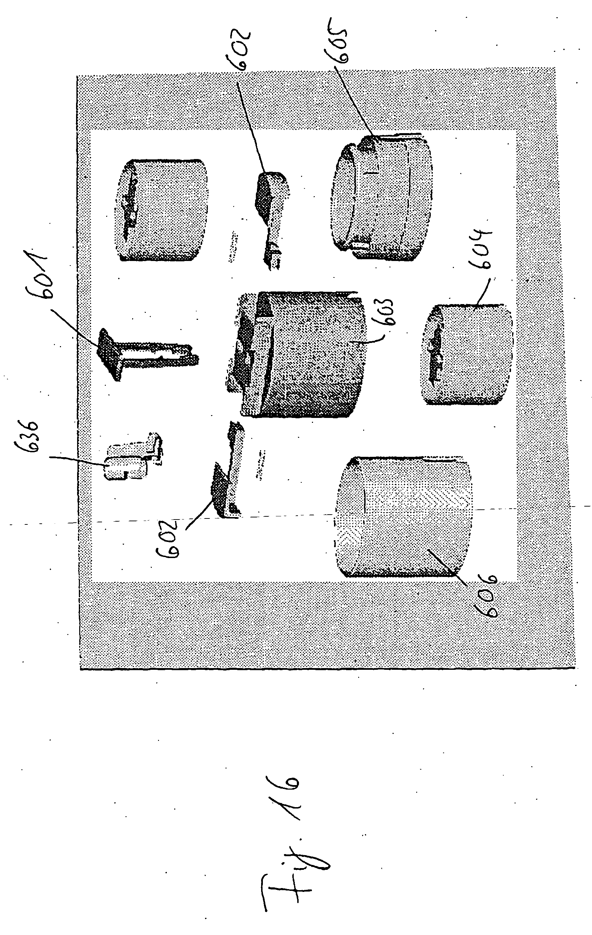

Fig. 16 zeigt Bestandteile der Insertionsvorrichtung der Fig. 13 bis 15. 606 zeigt die Hülse, 601 den Ladegriff, 602 die Auslöser, 636 einen Schutz für die Nadel, 604 das Vortriebsendglied, 605 das Elastomerteil und 603 das Gehäuse.

## Patentansprüche

1. Insertionsvorrichtung für Infusionssets (30, 230; 330; 632) mit einem Gehäuse (202; 302; 462; 502; 603) zum Umgeben eines in der Insertionsvorrichtung anbringbaren Infusionssets (230), mit einer Vortriebseinrichtung (5-8, 51, 61, 52, 62, 71, 81, 91, 92, 9; 205 - 208, 213, 241 - 245; 313; 401, 402, 405 - 423, 425 - 448; 513, 514, 505; 605, 604) zum Bewegen des Infusionssets in eine Vortriebsrichtung, wobei die Vortriebseinrichtung eine Vortriebsmechanik aufweist, die mit dem Gehäuse verbunden ist, zumindest ein elastisches Mittel (13, 213, 313, 513, 605) aufweist und ein Vortriebsendglied (9, 224, 335, 424, 505, 604) aufweist, das ausgebildet ist, ein Infusionsset in Vortriebsrichtung zu bewegen, wobei das Vortriebsendglied relativ zum Gehäuse mittels des elastischen Mittels so vorspannbar ist, dass das Vortriebsendglied bei Lösen der Vorspannung in die Vortriebsrichtung bewegt wird, wobei die Vortriebseinrichtung so ausgebildet ist, dass die Vortriebsbewegung bezüglich des Gehäuses gleitfrei erfolgt, indem die Vortriebseinrichtung eine Hebelmechanik aufweist, über die das Vortriebsendglied relativ zum Gehäuse bewegt wird und welche so gestaltet ist, dass die sich in Vortriebsrichtung bewegenden Teile der Hebelmechanik vom Gehäuse beabstandet sind oder zumindest keine Gleitbewegung bezüglich des Gehäuses durchführen, **dadurch gekennzeichnet, dass** das Gehäuse einen Infusionsset-Halter (235) aufweist, der zum Anbringen eines Infusionssets ausgebildet ist, wobei der Halter so ausgebildet und angeordnet ist, dass das Vortriebsendglied bei Bewegung in Vortriebsrichtung ein angebrachtes Infusionset aus der Halterung schlägt und die Vortriebseinrichtung so ausgebildet ist, dass im vorgespannten Zustand kein Kontakt zwischen angebrachtem Infusionsset und Vortriebsendglied besteht.

2. Insertionsvorrichtung nach Anspruch 1, **dadurch gekennzeichnet, dass** die Hebelmechanik Schenkelhebel (5, 6, 7, 8; 205 - 208; 405, 406, 415, 416; 505) aufweist, die drehbar über Drehverbindungsglieder verbunden sind, wobei zumindest ein Teil der Drehverbindungsglieder in Vortriebsrichtung bewegbar ist.

3. Insertionsvorrichtung nach Anspruch 2, **dadurch gekennzeichnet, dass** zumindest zwei erste sich gegensinnig drehende Drehverbindungsglieder (71, 81, 91, 92; 241, 247, 246, 248) in Vortriebsrichtung bewegbar sind und ihre Vortriebsbewegung durch die verkoppelte Drehbewegung zweier zweiter ortsfester Drehverbindungsglieder (51, 61; 242, 245) synchronisiert ist, wobei jeweils eines der ersten Drehverbindungsglieder mit einem der zweiten Drehverbindungsglieder über einen Schenkelhebel verbunden ist.

4. Insertionsvorrichtung nach Anspruch 1, **dadurch gekennzeichnet, dass** die Hebelmechanik (401, 402, 405, 406, 421, 422, 411, 412, 415 - 418, 421 - 423, 425, 447, 448) zumindest seitlich (401, 411, 402, 412) der Vortriebsrichtung am Gehäuse angelenkt ist, um das Vortriebsendglied in Vortriebsrichtung zu führen.

5. Insertionsvorrichtung nach Anspruch 4, **dadurch gekennzeichnet, dass** die Hebelmechanik eine Lemniskatenführung umfasst, die das Vortriebsglied in Vortriebsrichtung führt.

6. Insertionsvorrichtung nach Anspruch 4, **dadurch gekennzeichnet, dass** die Hebelmechanik zumindest an einer Stelle (401, 411, 402, 412; 514) an das Gehäuse angelenkt ist und das Vortriebsendglied über zumindest einen Schenkelhebel (406, 405, 415, 416, 505) um diese Stelle bezüglich des Gehäuses kontaktlos rotiert.

7. Insertionsvorrichtung nach Anspruch 1, **dadurch gekennzeichnet, dass** das elastische Mittel (605) das Gehäuse (603) und das Vortriebsendglied (604) so verbindet, dass es elastisch gedehnt wird, wenn das Vortriebsendglied entgegen der Vortriebsrichtung bewegt wird.

8. Insertionsvorrichtung nach Anspruch 7, **dadurch gekennzeichnet, dass** das elastische Mittel mehrere dehnbare Bänder (605b) umfasst, die symmetrisch um das Vortriebsendglied herum angeordnet sind, um das Vortriebsendglied auf einem vorgesehenen Bewegungspfad in Vortriebsrichtung zu halten, wobei das Vortriebsendglied auf dem Bewegungspfad vom Gehäuse beabstandet bleibt.

9. Insertionsvorrichtung nach Anspruch 1, mit mindestens einem Drehverbindungsglied (542) an einer Seitenwand des Gehäuses, mit mindestens einem Schenkelhebel (505), der mit einem ersten Ende an dem mindestens einen Drehverbindungsglied verbunden ist, wobei der Schenkelhebel durch ein elastisches Glied (513) gegen die Seitenwand vorspannbar ist und das zweite, zu dem ersten entgegengesetzt liegende Ende des Schenkelhebels so ausgebildet ist, dass es ein Infusionsset aus einer Halterung schlagen kann und wobei der Schenkelhebel um das mindestens eine Drehverbindungsglied geschwenkt wird, wenn das elastische Mittel durch einen Auslösemechanismus entspannt wird.

## Claims

1. Insertion device for infusion sets (30, 230; 330; 632) with a housing (202; 302; 462; 502; 603) for enclosing an infusion set (230) which can be fitted in the insertion device, with an advancing means (5-8, 51, 61, 52, 62, 71, 81, 91, 92, 9; 205 - 208, 213, 241 - 245; 313; 401, 402, 405 - 423, 425 - 448; 513, 514, 505; 605, 604) for moving the infusion set in direction of advance, wherein the advancing means has an advancing mechanism which is connected to the housing, has at least one elastic means (13, 213, 313, 513, 605) and has an advancing end-member (9, 224, 335, 424, 505, 604) which is formed to move an infusion set in direction of advance, wherein the advancing end-member can be pre-stressed relative to the housing by means of the elastic means such that the advancing end-member is moved in the direction of advance upon the release of the initial stress, wherein the advancing means is formed such that the advance movement takes place slide-free relative to the housing, the advancing means having a lever mechanism via which the advancing end-member is moved relative to the housing and which is designed such that the parts of the lever mechanism moving in direction of advance are kept at a distance from the housing or at least do not perform a sliding movement relative to the housing, **characterized in that** the housing has an infusion set holder (235) which is formed for the fitting of an infusion set, wherein the holder is formed and arranged such that the advancing end-member, upon movement in direction of advance, strikes a fitted infusion from the holder and the advancing means is formed such that in pre-stressed state there is no contact between the fitted infusion set and advancing end-member.

2. Insertion device according to claim 1, **characterized in that** the lever mechanism has lever arms (5, 6, 7, 8; 205 - 208; 405, 406, 415, 416; 505) which are connected rotatable via rotary connecting members, wherein at least part of the rotary connecting members can be moved in direction of advance.

3. Insertion device according to claim 2, **characterized in that** at least two first rotary connecting members (71, 81, 91, 92; 241, 247, 246, 248) rotating in opposite directions can be moved in direction of advance and their advance movement is synchronised by the coupled rotary movement of two second stationary rotary connecting members (51, 61; 242, 245), wherein in each case one of the first rotary connecting members is connected to one of the second rotary connecting members via a lever arm.

4. Insertion device according to claim 1, **characterized in that** the lever mechanism (401, 402, 405, 406, 421, 422, 411, 412, 415 - 418, 421 - 423, 425, 447, 448) is articulated to the housing at least laterally (401, 411, 402, 412) to the direction of advance in order to guide the advancing end-member in direction of advance.

5. Insertion device according to claim 4, **characterized in that** the lever mechanism includes a lemniscate guide which guides the advancing end-member in direction of advance.

6. Insertion device according to claim 4, **characterized in that** the lever mechanism is articulated to the housing at at least one point (401, 411, 402, 412; 514) and the advancing end-member rotates contactlessly about this point relative to the housing via at least one lever arm (406, 405, 415, 416, 505).

7. Insertion device according to claim 1, **characterized in that** the elastic means (605) connects the housing (603) and the advancing end-member (604) such that it is extended elastically when the advancing end-member is moved against the direction of advance.

8. Insertion device according to claim 7, **characterized in that** the elastic means comprises several extendable bands (605b), which are arranged symmetrically around the advancing end-member, in order to keep the advancing end-member on a given path of movement in direction of advance, wherein the advancing end-member remains kept at a distance from the housing on the path of movement.

9. Insertion device according to claim 1, with at least one rotary connecting member (542) on a side wall of the housing, with at least one lever arm (505) which is connected to a first end on the at least one rotary connecting member, wherein the lever arm can be pre-stressed against the side wall by an elastic member (513) and the second end, lying opposite the first, of the lever arm is formed such that it can strike an infusion set from a holder and wherein the lever arm is pivoted around the at least one rotary connecting member when the elastic means is relieved by a trigger mechanism.

## Revendications

1. Dispositif d'insertion pour dispositifs de perforation (30, 230 ; 330 ; 632) avec un boîtier (202, 302 ; 462 ; 502 ; 603) pour entourer un dispositif de perforation (230) pouvant être mis en place dans le dispositif d'insertion, avec un dispositif d'avance (5 - 8, 51, 61, 52, 62, 71, 81, 91, 92, 9 ; 205 - 208,213,241 - 245 ; 313 ; 401, 402, 405 - 423, 425 - 448 ; 513, 514, 505 ; 605, 604) pour déplacer le dispositif de perforation dans un sens d'avance, le dispositif d'avance comportant une mécanique d'avance qui est reliée au boîtier, au moins un moyen élastique (13, 213, 313, 513, 605), et un élément terminal d'avance (9, 224, 335, 424, 505, 604) réalisé pour déplacer un dispositif de perforation dans le sens d'avance, l'élément terminal d'avance pouvant être précontraint par rapport au boîtier grâce au moyen élastique de telle manière que l'élément terminal d'avance est déplacé dans le sens d'avance lorsque la précontrainte est relâchée, le dispositif d'avance étant réalisé de manière à ce que le mouvement d'avance par rapport au boîtier se fasse sans glissement, le dispositif d'avance comportant une mécanique à levier par le biais de laquelle l'élément terminal d'avance est déplacé par rapport au boîtier et configurée de telle manière que les éléments de la mécanique à levier qui se déplacent dans le sens d'avance sont écartés du boîtier ou, du moins, n'effectuent pas de mouvement de glissement par rapport au boîtier, **caractérisé en ce que** le boîtier comporte un support de dispositif de perforation (235) qui est réalisé pour la mise en place d'un dispositif de perforation, le support étant réalisé et disposé de telle manière qu'en cas de mouvement dans le sens d'avance l'élément terminal d'avance éjecte un dispositif de perforation en place du support et le dispositif d'avance étant réalisé de telle manière qu'à l'état précontraint il n'y a pas de contact entre le dispositif de perforation en place et l'élément terminal d'avance.

2. Dispositif d'insertion selon la revendication 1, **caractérisé en ce que** la mécanique à levier comporte des leviers à branche (5, 6, 7, 8 ; 205 - 208 ; 405, 406, 415, 416 ; 505) qui sont reliés de manière à pouvoir tourner via des éléments à joint tournant, au moins une partie des éléments à joint tournant pouvant être déplacée dans le sens d'avance.

3. Dispositif d'insertion selon la revendication 2, **caractérisé en ce qu'**au moins deux premiers éléments à joint tournant (71, 81, 91, 92 ; 241, 247, 246, 248) qui tournent en sens inverse l'un par rapport à l'autre peuvent être déplacés dans le sens d'avance et **en ce que** leur mouvement d'avance est synchronisé par le mouvement de rotation couplé de deux seconds éléments à joint tournant fixes (51, 61 ; 242, 245), chacun des premiers éléments à joint tournant étant relié à un des seconds éléments à joint tournant via un levier à branche.

4. Dispositif d'insertion selon la revendication 1, **caractérisé en ce que** la mécanique à levier (401, 402, 405, 406, 421, 422, 411, 412, 415 - 418, 421 - 423, 425, 447, 448) est articulée au boîtier au moins latéralement (401, 411, 402, 412) par rapport au sens d'avance, afin de guider l'élément terminal d'avance dans le sens d'avance.

5. Dispositif d'insertion selon la revendication 4, **caractérisé en ce que** la mécanique à levier comprend un guidage à lemniscate qui guide l'élément d'avance dans le sens d'avance.

6. Dispositif d'insertion selon la revendication 4, **caractérisé en ce que** la mécanique à levier est articulée au boîtier au moins à un endroit (401, 411, 402, 412 ; 514) et **en ce que** l'élément terminal d'avance tourne via au moins un levier à branche (406, 405, 415, 416, 505) autour de ce point sans contact avec le boîtier.

7. Dispositif d'insertion selon la revendication 1, **caractérisé en ce que** le moyen élastique (605) relie le boîtier (603) et l'élément terminal d'avance (604) de telle manière qu'il est allongé de façon élastique lorsque l'élément terminal d'avance est déplacé dans le sens contraire au sens d'avance.

8. Dispositif d'insertion selon la revendication 7, **caractérisé en ce que** le moyen élastique comprend plusieurs bandes extensibles (605b) qui sont disposées de manière symétrique autour de l'élément terminal d'avance afin de maintenir l'élément terminal d'avance dans le sens d'avance sur l'itinéraire d'avance prévu, l'élément terminal d'avance restant écarté du boîtier sur l'itinéraire d'avance.

9. Dispositif d'insertion selon la revendication 1, avec au moins un élément à joint tournant (542) sur une paroi latérale du boîtier, avec au moins un levier à branche (505) qui est relié avec une première extrémité à un élément à joint tournant minimum, le levier à branche pouvant être précontraint contre la paroi latérale par un élément élastique (513) et la deuxième extrémité du levier à branche, située à l'opposé de la première, étant réalisée de telle manière qu'elle peut accueillir un dispositif de perforation ou l'éjecter d'un support et le levier à branche pivotant autour de l'élément à joint tournant minimum lorsque le moyen élastique est relâché par un mécanisme de déclenchement.
